(19) Europäisches Patentamt
European Patent Office
Office européen des brevets

(11) Veröffentlichungsnummer: **0 422 461 A2**

(12) **EUROPÄISCHE PATENTANMELDUNG**

(21) Anmeldenummer: **90118641.1**

(51) Int. Cl.5: **C07D 251/30**, A01N 43/64

(22) Anmeldetag: **28.09.90**

(30) Priorität: **11.10.89 DE 3933933**

(43) Veröffentlichungstag der Anmeldung:
**17.04.91 Patentblatt 91/16**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Uhr, Hermann, Dr.**
**Düsseldorfer Strasse 67**
**W-5090 Leverkusen 3(DE)**
Erfinder: **Adler, Alfons, Dr.**
**Hahnenweg 8**
**W-5000 Köln 80(DE)**
Erfinder: **Widdig, Arno, Dr.**
**Eifgenstrasse 8**
**W-5068 Odenthal(DE)**
Erfinder: **Hagemann, Hermann, Dr.**
**Kandinskystrasse 52**
**W-5090 Leverkusen 1(DE)**
Erfinder: **Hänssler, Gerd, Dr.**
**Am Arenzberg 58 a**
**W-5090 Leverkusen 3(DE)**

(54) **Trisubstituierte 1,3,5-Triazin-2,4,6-trione.**

(57) Trisubstituierte 1,3,5-Triazin-2,4,6-trione der Formel (I)

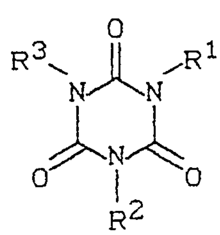

(I)

in welcher
$R^1$, $R^2$ und $R^3$ verschieden sind und die in der Beschreibung gegebenen Bedeutungen haben und ihre Verwendung zur Schädlingsbekämpfung.

Die neuen Verbindungen sind durch die Formel (I) definiert und sie können nach Analogieverfahren hergestellt werden, z.B. indem man geeignete 1,3-disubstituierte 1,3,5-Triazin-2,4,6-trione mit geeigneten Verbindungen der Formel (II) umsetzt oder geeignete Harnstoffderivate mit geeigneten Bis-chlor-carbonylaminen umsetzt. Einige der Ausgangsprodukte der Harnstoffe sind auch neu und können aus geeigneten Isocyanaten mit Aminen hergestellt werden.

EP 0 422 461 A2

# TRISUBSTITUIERTE 1,3,5-TRIAZIN-2,4,6-TRIONE

Die Erfindung betrifft neue trisubstituierte 1,3,5-Triazin-2,4,6-trione, Verfahren zu ihrer Herstellung und ihre Verwendung zur Schädlingsbekämpfung, vor allem als Fungizide.

Es ist bereits bekannt geworden, daß 2-Arylamino-4,6-dichlor-s-triazine, wie z.B. das 4,6-Dichlor-N-(2-chlor-phenyl)-1,3,5-triazin-2-amin, fungizide Eigenschaften besitzen (vgl. DAS 1 670 675). Die selektive fungizide Wirksamkeit dieser Stoffe ist jedoch nur auf wenige Pilze beschränkt und nicht immer ausreichend.

Weiterhin sind trisubstituierte 1,3,5-Triazin-2,4,6-trione, die auf jeden Fall einen Arylrest tragen, als Fungizide bekannt (vgl. DE-OS 3 618 662) und auch Verbindungen, die auf jeden Fall ein heterocyclisch anelliertes Phenyl tragen, bekannt (vgl. DE-OS 3 810 080).

Die Wirksamkeit dieser vorbekannten Verbindungen ist jedooh insbesondere bei niedrigen Aufwandmengen und Konzentrationen nicht in allen Anwendungsgebieten völlig zufriedenstellend.

Es wurden neue trisubstituierte 1,3,5-Triazin-2,4,6-trione der allgemeinen Formel (I)

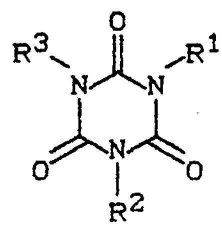

(I)

in welcher
$R^1$, $R^2$ und $R^3$ unterschiedliche Reste sind und
$R^1$ für einen gegebenenfalls substituierten aliphatischen oder cycloaliphatischen Rest steht,
$R^2$ für einen gegebenenfalls substituierten aliphatischen Rest steht und
$R^3$ für ein gegebenenfalls substituiertes Aralkyl steht,
gefunden.

Weiterhin wurde gefunden, daß man die neuen trisubstituierten 1,3,5-Triazin-2,4,6-trione der Formel (I), in welcher
$R^1$, $R^2$ und $R^3$ unterschiedliche Reste sind und
$R^1$ für einen gegebenenfalls substituierten aliphatischen oder cycloaliphatischen Rest steht,
$R^2$ für einen gegebenenfalls substituierten aliphatischen Rest steht und
$R^3$ für ein gegebenenfalls substituiertes Aralkyl steht, erhält,
wenn man entweder

a) 1,3-disubstituierte 1,3,5-Triazin-2,4,6-trione der allgemeinen Formel (II)

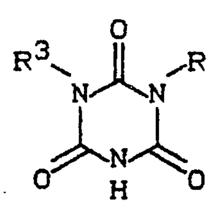

(II)

in welcher
$R^1$ und $R^3$ die oben angegebenen Bedeutungen haben, mit Verbindungen der allgemeinen Formel (III)
$R^2$-X (III)
in welcher
$R^2$ die oben angegebene Bedeutung hat und
X eine Abgangsgruppe, wie z.B. Halogen, Sulfat, Mesylat oder Tosylat bedeutet,
gegebenenfalls in Gegenwart eines Verdünnungs- oder Lösungsmittel und gegebenenfalls in Gegenwart eines Säurebindemittels umsetzt, oder wenn man

b) 1,3-disubstituierte 1,3,5-Triazin-2,4,6-trione der allgemeinen Formel (IV)

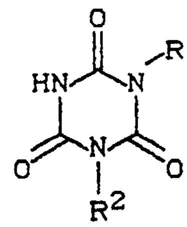

(IV)

in welcher
$R^1$ und $R^2$ die oben angegebenen Bedeutungen haben, mit Verbindungen der allgemeinen Formel (V)
$R^3$-X (V)
in welcher
$R^3$ und X die oben angegebene Bedeutung haben,
gegebenenfalls in Gegenwart eines Verdünnungs- oder Lösungsmittels umsetzt oder wenn man
c) N,N′-disubstituierte Harnstoffe der allgemeinen Formel (VI)
$R^1$-NH-CO-NH-$R^3$ (VI)
in welcher
$R^1$ und $R^3$ die oben angegebenen Bedeutungen haben, mit einem Bischlorcarbonylamin der allgemeinen Formel (VII)

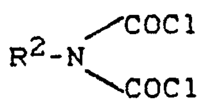

(VII)

in welcher
$R^2$ die oben angegebenen Bedeutung hat,
gegebenenfalls in Gegenwart eines Verdünnungs- oder Lösungsmittel und gegebenenfalls in Gegenwart eines Säurebindemittels umsetzt oder wenn man
d) N,N′-disubstituierte Harnstoffe der allgemeinen Formel (VIII)
$R^1$-NH-CO-NH-$R^2$ (VIII)
in welcher
$R^1$ und $R^2$ die oben angegebene Bedeutung haben, mit Verbindungen der allgemeinen Formel (IX)

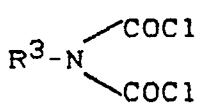

(IX)

in welcher
$R^3$ die oben angegebene Bedeutung hat, gegebenenfalls in Gegenwart eines Verdünnungs- oder Lösungsmittels und gegebenenfalls in Gegenwart eines Säurebindemittels, umsetzt.

Schließlich wurde gefunden, daß die neuen trisubstituierten 1,3,5-Triazin-2,4,6-trione der Formel (I) sehr gute biologische Eigenschaften besitzen und zur Bekämpfung von Schädlingen, insbesondere von Pilzen und vor allem zur selektiven Bekämpfung von Schadpilzen im Reis geeignet sind. Es ist als äußerst überraschend zu bezeichnen, das die erfindungsgemäßen Stoffe eine bessere Wirksamkeit zeigen als bekannte Verbindungen aus dem Stand der Technik gleicher Wirkungsrichtung und/oder konstitutionell ähnlichen Verbindungen.

Die erfindungsgemäßen trisubstituierten 1,3,5-Triazin-2,4,6-trione sind durch die Formel (I) allgemein definiert. Bevorzugt sind Verbindungen der Formel (I),
in welcher
$R^1$, $R^2$ und $R^3$ unterschiedliche Reste sind und
$R^1$ für geradkettiges oder verzweigtes Alkyl mit 1 bis 12 Kohlenstoffatomen, geradkettiges oder verzweigtes Alkenyl mit 3 bis 12 Kohlenstoffatomen oder geradkettiges oder verzweigtes Alkinyl mit 3 bis 12 Kohlenstoffatomen steht, welche gegebenenfalls ein- bis mehrfach, gleich oder verschieden substituiert sind durch Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 3 Kohlenstoffatomen, Halogenalkylthio mit 1 bis 3 Kohlenstoffatomen und 1 bis 5 gleichen oder verschiedenen Halogenatomen; weiterhin $R^1$ für Cycloalkyl mit

3 bis 8 Kohlenstoffatomen steht, welches gegebenenfalls ein- bis sechsfach, gleich oder verschieden substituiert ist durch Alkyl mit 1 bis 4 Kohlenstoffatomen, Alkoxy mit 1 bis 3 Kohlenstoffatomen, Halogenalkoxy mit 1 bis 3 Kohlenstoffatomen und 1 bis 5 gleichen oder verschiedenen Halogenatomen, Alkylthio mit 1 bis 3 Kohlenstoffatomen und Halogenalkylthio mit 1 bis 3 Kohlenstoffatomen und 1 bis 5 gleichen oder verschiedenen Halogenatomen,

$R^2$ für Alkyl mit 1 bis 10 Kohlenstoffatomen, Alkenyl mit 3 bis 10 Kohlenstoffatomen, Alkinyl mit 3 bis 10 Kohlenstoffatomen, Alkoxyalkyl mit je 1 bis 3 Kohlenstoffatomen im Alkoxyteil und im Alkylteil, Alkylthioalkyl mit je 1 bis 3 Kohlenstoffatomen im Alkylthioteil und im Alkylteil, Alkoxycarbonylalkyl mit 1 bis 3 Kohlenstoffatomen im Alkoxyteil und 2 oder 3 Kohlenstoffatomen im Alkylteil oder für Cyanoalkyl mit 1 bis 5 Kohlenstoffatomen im Alkylteil steht,

$R^3$ für Aralkyl mit 1 bis 12 Kohlenstoffatomen im geradkettigen oder verzweigten Alkylteil und 6 bis 12 Kohlenstoffatomen im Arylteil steht, wobei Aryl für Phenyl oder Naphthyl steht und die Ringe jeweils gegebenenfalls ein- bis fünffach, gleich oder verschieden substituiert sind durch Halogen, Alkyl mit 1 bis 6 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Monoalkyl- oder Dialkylamino mit gegebenenfalls gleichen oder verschiedenen, geradkettigen oder verzweigten Alkylresten mit je 1 bis 4 Kohlenstoffatomen, Nitro und/oder Benzyloxy oder der Phenylring gegebenenfalls einer ankondensierten, gesättigten Ring mit 3 bis 5 Kohlenstoffatomen tragen kann.

Besonders hervorzuheben sind Verbindungen der Formel (I),

in denen

$R^1$, $R^2$ und $R^3$ unterschiedliche Reste sind und

$R^1$ für geradkettiges oder verzweigtes Alkyl mit 1 bis 10 Kohlenstoffatomen, geradkettiges oder verzweigtes Alkenyl mit 3 bis 8 Kohlenstoffatomen oder geradkettiges oder verzweigtes Alkinyl mit 3 bis 8 Kohlenstoffatomen steht, welche jeweils gegebenenfalls ein- bis dreifach, gleich oder verschieden substituiert sind durch Alkoxy mit 1 bis 3 Kohlenstoffatomen, Halogenalkoxy mit 1 oder 2 Kohlenstoffatomen und 1 bis 3 Chlor- und/oder Fluoratomen, Alkylthio mit 1 oder 2 Kohlenstoffatomen, Halogenalkylthio mit 1 oder 2 Kohlenstoffatomen und 1 bis 3 Chlor- und/oder Fluoratomen; $R^1$ weiterhin für Cycloalkyl mit 3 bis 6 Kohlenstoffatomen steht, welches gegebenenfalls ein- bis sechsfach, gleich oder verschieden substituiert ist durch Fluor, Chlor, Alkyl mit 1 bis 3 Kohlenstoffatomen, Alkoxy mit 1 oder 2 Kohlenstoffatomen, Halogenalkoxy mit 1 oder 2 Kohlenstoffatomen und 1 bis 5 Chlor-und/oder Fluoratomen, Alkylthio mit 1 oder 2 Kohlenstoffatomen und Halogenalkylthio mit 1 oder 2 Kohlenstoffatomen und 1 bis 3 Chlor- und/oder Fluoratomen,

$R^2$ für Alkyl mit 1 bis 8 Kohlenstoffatomen, Alkenyl mit 3 bis 8 Kohlenstoffatomen, Alkinyl mit 3 bis 8 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht und

$R^3$ für Aralkyl mit 1 bis 9 Kohlenstoffatomen im geradkettigen oder verzweigten Alkylteil und Aryl für Phenyl oder Naphthyl steht, welche gegebenenfalls ein- bis vierfach, gleich oder verschieden substituiert sein können durch Fluor, Chlor, Brom, Alkyl mit 1 bis 5 Kohlenstoffatomen, Halogenalkyl mit 1 oder 2 Kohlenstoffatomen und 1 bis 5 Chlor- und/oder Fluoratomen, Alkoxy mit 1 bis 3 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und 1 bis 5 Chlor- und/oder Fluoratomen, Dialkylamino mit gleichen oder verschiedenen, geradkettigen oder verzweigten Alkylresten mit je 1 bis 3 Kohlenstoffatomen, Nitro und/oder Benzyloxy oder der Phenylring in 1,2-Stellung eine Tri- oder Tetramethylenkette enthält.

Ganz besonders hervorzuheben sind Verbindungen der Formel (I),

in denen

$R^1$, $R^2$ und $R^3$ unterschiedliche Reste sind und

$R^1$ für geradkettiges oder verzweigtes Alkyl mit 1 bis 8 Kohlenstoffatomen, geradkettiges oder verzweigtes Alkenyl mit 3 bis 6 Kohlenstoffatomen oder geradkettiges oder verzweigtes Alkinyl mit 3 bis 6 Kohlenstoffatomen steht, welche ein- bis dreifach, gleich oder verschieden substituiert sein können durch Alkoxy mit 1 oder 2 Kohlenstoffatomen, Alkylthio mit 1 oder 2 Kohlenstoffatomen; oder $R^1$ weiterhin für Cycloalkyl mit 3 bis 6 Kohlenstoffatomen steht, welches gegebenenfalls ein- bis dreifach, gleich oder verschieden durch Fluor, Chlor, Methyl, Ethyl, Methoxy, Ethoxy, Methylthio, Ethylthio und Trifluormethyl substituiert sein kann,

$R^2$ für Methyl, Ethyl, Propyl, n-, sec.-, iso-, tert.-Butyl, n-Pentyl, iso-Pentyl, neo-Pentyl, tert.-Pentyl, Allyl, 2-Butenyl, Propargyl, 2-Butinyl, Methoxymethyl, Ethoxymethyl, Methylthiomethyl, Methoxycarbonylmethyl, Ethoxycarbonylmethyl, Methoxycarbonylethyl, Cyanmethyl oder Cyanethyl steht und

$R^3$ für Aralkyl mit 1 bis 7 Kohlenstoffatomen im geradkettigen oder verzweigten Alkylrest und Aryl für Phenyl oder Naphthyl steht, welche ein- bis dreifach, gleich oder verschieden substituiert sein können durch Fluor, Chlor, Alkyl mit 1 bis 4 Kohlenstoffatomen, Halogenalkyl mit 1 oder 2 Kohlenstoffatomen und 1 bis 5 Chlor- und/ oder Fluoratomen, Alkoxy mit 1 oder 2 Kohlenstoffatomen, Halogenalkoxy mit 1 oder 2 Kohlenstoffatomen und 1 bis 5 Chlor- und/oder Fluoratomen, Benzyloxy oder der Phenylring in 1,2-Stellung eine Trimethylenkette trägt.

Verwendet man gemäß Verfahren a) 1-tert.-Butyl-3-[2-(4-methylphenyl)-ethyl]-1,3,5-triazin -2,4,6-trion und Ethyliodid als Ausgangsstoffe, so kann der Reaktionsverlauf durch das folgende Formelschema veranschaulicht werden:

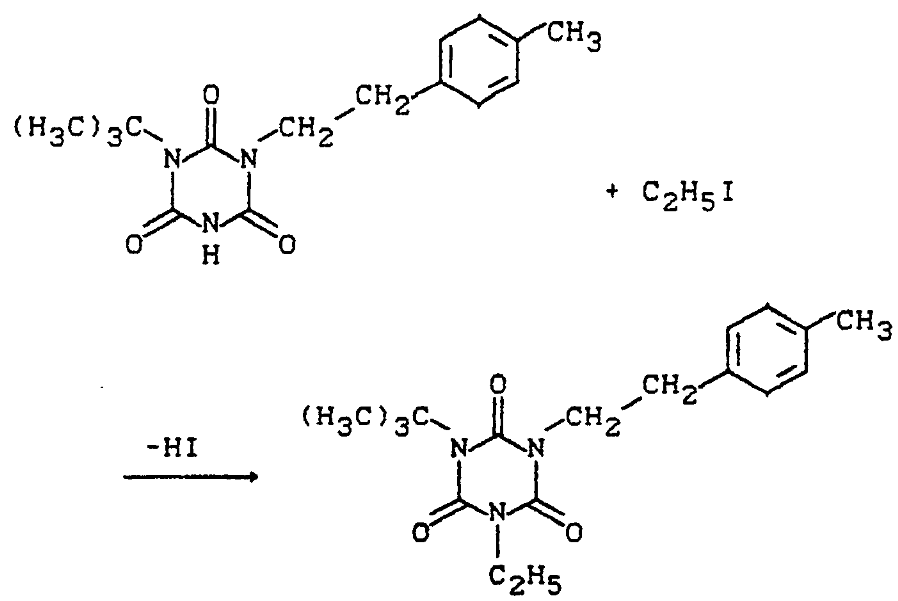


Verwendet man gemäß Verfahren b) 1-tert.-Butyl-3-ethyl-1,3,5-triazin-2,4,6-trion und Benzylbromid als Ausgangsstoffe, so kann der Reaktionsverlauf durch das folgende Reaktionsschema veranschaulicht werden:

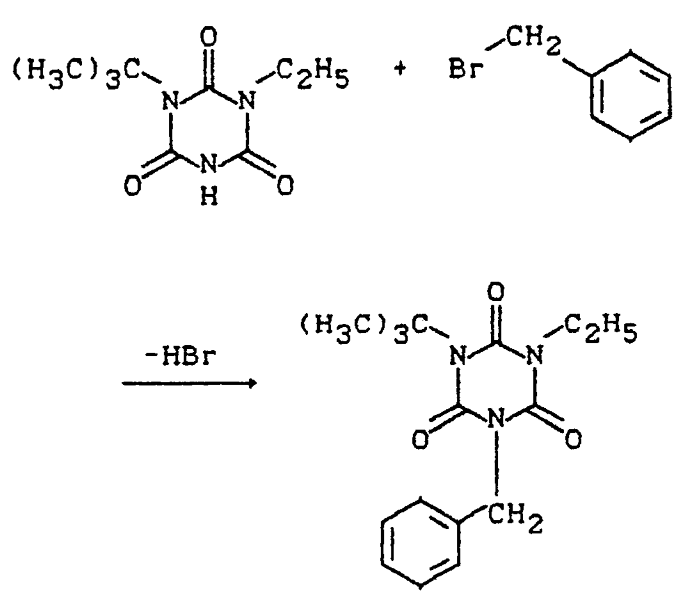


Verwendet man gemäß Verfahren c) N-[2-(4-Methoxyphenyl)-2-(2-propyl)-ethyl]-N'-(neopentyl)-harnstoff

und Bischlorcarbonyl-N-ethylamin als Ausgangsstoffe, so kann der Reaktionsverlauf durch das folgende Formelschema veranschaulicht werden:

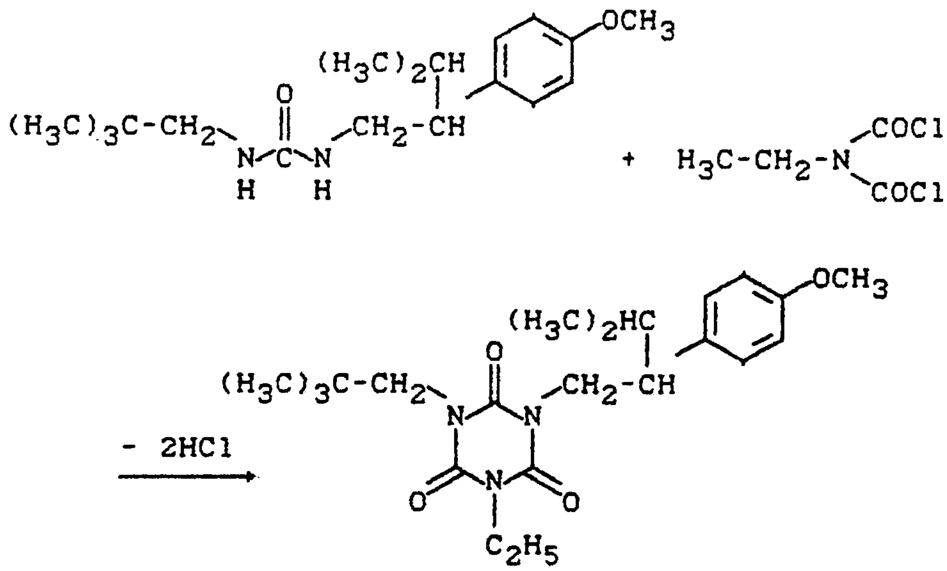


Verwendet man gemäß Verfahren d) Bis-chlorcarbonyl-N-[2-(2,6-dichlor-phenyl)-ethylamin und N-Ethyl-N'-(2,2-dimethyl-propyl)-harnstoff als Ausgangsstoffe, so kann der Reaktionsverlauf durch das folgende Formelschema veranschaulicht werden:

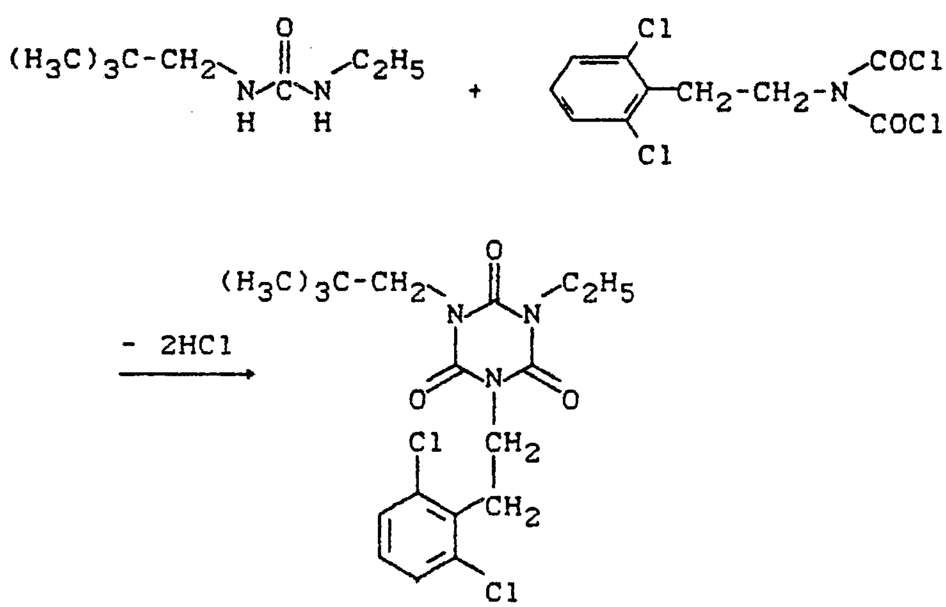


Die bei den erfindungsgemäßen Verfahren a) und b) als Ausgangsverbindungen benötigten disubstituierten 1,3,5-Triazin-2,4,6-trione der allgemeinen Formeln (II) und (IV) sind teilweise bekannt und können in an sich bekannter Weise z.B aus N,N'-disubstituierten Harnstoffen und Chlorcarbonylisocyanat erhalten werden (vgl. Angew. Chem. 89 , 789 (1977)). Die für die Verfahren a) und b) zu verwendenden Alkylierungsmittel der Formeln (III) und (V) sind ebenfalls bekannt. Bevorzugt steht hierbei X für Chlorid, Bromid, Iodid, Sulfat, Mesylat oder Tosylat.

Die für die erfindungsgemäßen Verfahren c) und d) zu verwendenden Harnstoffe der allgemeinen Formeln (VI) und (VIII) sind größtenteils bekannt und durch Addition von geeigneten Isocyanaten an primäre Amine zugänglich (vgl. Houben-Weyl: Methoden der organischen Chemie, Band E4, (1983), S.352, Thieme-Verlag, Stuttgart).

Die bei Verfahren c) und d) verwendeten Bis-chlorcarbonylamine der allgemeinen Formel (VII) und (IX) sind ebenfalls bekannt (vgl. Houben-Weyl: Methoden der organischen Chemie, Band E4, (1983), S. 1022, Thieme-Verlag, Stuttgart).

Die Reaktionstemperaturen können bei den erfindungsgemäßen Verfahren in einem größeren Temperaturbereich variiert werden. Im allgemeinen arbeitet man bei Verfahren a) und b) zwischen 20°C und 150°C, vorzugsweise zwischen 50°C und 120°C, bei Verfahren c) und d) im allgemeinen zwischen 0°C und 150°C, vorzugsweise zwischen 20°C und 120°C.

Bei der Durchführung der erfindungsgemäßen Verfahren setzt man die Ausgangsstoffe und gegebenenfalls die Säurebindemittel in etwa äquimolaren Mengen ein. Ein Überschuß an säurebindenden Mitteln schadet im allgemeinen nicht.

Die Umsetzungen werden bevorzugt in Gegenwart eines Verdünnungsmittels durchgeführt. Als Verdünnungsmittel kommen alle inerten organischen Lösungsmittel in Frage. Hierzu gehören vorzugsweise Kohlenwasserstoffe wie Toluol und Xylol; chlorierte Kohlenwasserstoffe wie Chlorbenzol und Chloroform; Ketone wie Aceton; Ether wie Tetrahydrofuran und Dioxan; Nitrile wie Acetonitril.

Als Säurebinder können alle üblichen säurebindenden Mittel verwendet werden. Hierzu gehören vorzugsweise tertiäre Amine wie Triethylamin und Pyridin; Alkalihydroxide wie Natrium- und Kaliumhydroxid und Alkalicarbonate und -hydrogencarbonate wie Kaliumcarbonat und Natriumhydrogencarbonat.

Die Aufarbeitung und Isolierung der erfindungsgemäßen Verbindungen erfolgt in üblicher Weise. Sie fallen entweder sofort kristallin an oder bleiben als Kristallisat oder Öl nach Verdampfen des Lösungsmittels zurück.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch z.B. als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis; Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis; Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in üblichen Formulierungen überführt werden, wie Lösungen, Emulsionen, Suspensionen, Pulver, Schäume, Pasten, Granulate, Aerosole, Feinstverkapselungen in polymeren Stoffen

und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektiziden, Akarizide und in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ulra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel A (Verfahren a) (entspricht Beispiel-Nummer 38)

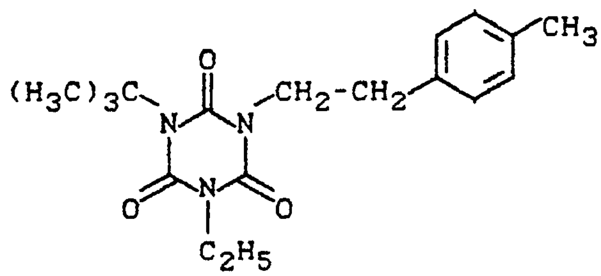

Zu einer Lösung aus 10 g (33,0 mMol) 1-tert.-Butyl-3-[2-(4-methylphenyl)-ethyl]-1,3,4,-triazin-2,4,6-trion und 9,1 g (66 mMol) Kaliumcarbonat in 50 ml absolutem Acetonitril werden bei Raumtemperatur 6,2 g (39,6 mMol) Ethyliodid getropft. Das Reaktionsgemisch wird 5 h unter Rückfluß gekocht. Nach dem Abkühlen werden die festen Bestandteile abgetrennt und die Reaktionslösung in kaltes Wasser gegeben. Die ausgefallenen Kristalle werden abgesaugt und aus Wasser/Methanol umkristallisiert. Man erhält nach dem Trocknen 9,5 g (87 % der Theorie) 1-tert.-Butyl-5-ethyl-3-[2-(4-methylphenyl)-ethyl]-1,3,5-triazin-2,4,6-trion in Form von Kristallen mit einem Schmelzpunkt von 64° C.

Beispiel B (Verfahren b) (entspricht Beispiel-Nummer 1)

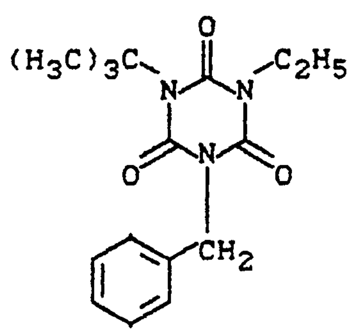

Zu einer Lösung aus 10 g (46,9 mMol) 1-tert.-Butyl-3-ethyl-1,3,5-triazin-2,4,6-trion und 9,9 g (93 mMol) Natriumcarbonat in 50 ml absolutem Aceton werden bei Raumtemperatur 7,7 g (45 mMol) Benzylbromid getropft. Das Reaktionsgemisch wird 6 h unter Rückfluß gekocht. Nach dem Abkühlen werden die festen Bestandteile abgetrennt und die Reaktionslösung eingeengt. Der Rückstand wird in Methylenchlorid aufgenommen und zweimal mit Wasser gewaschen. Nach dem Trocknen der organischen Phase über Natriumsulfat wird eingeengt und im Vakuum destilliert. Man erhält 8,5 g (60 % der Theorie) 5-Benzyl-1-tert.-butyl-3-ethyl-1,3,5-triazin-2,4,6-trion als dickflüssiges Öl mit einem Siedepunkt von 170° C/0,03 mbar.

Beispiel C (Verfahren c) (entspricht Beispiel-Nummer 53)

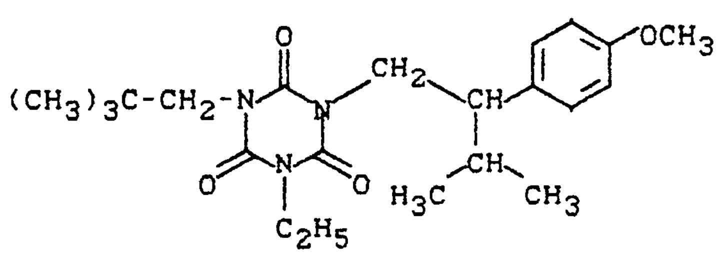

3 g (0,01 Mol) N-[2-(4-Methoxy-phenyl)-2-isopropyl]-ethyl-N′-(neopentyl)-harnstoff werden in 50 ml absolutem Dioxan gelöst und mit 4,2 ml (0,03 Mol) Triethylamin versetzt. Bei Raumtemperatur tropft man langsam eine Lösung von 1,6 g (0,01 Mol) Bischlorcarbonyl-N-ethylamin in 20 ml Dioxan zu. Man rührt 1 h

bei Raumtemperatur und dann noch 15 Minuten unter Rückfluß. Nach dem Abkühlen wird auf Eiswasser gegeben und mit Methylenchlorid extrahiert. Nach zweifachem Waschen der Methylenchlorid-Phase mit Wasser, trocknet man über Natriumsulfat und dampft ein.
Ausbeute: 2,1 g = 51 % der Theorie.
Phys. Daten siehe Beispiel 53.

Analog den angegebenen Beispielen und/oder den im Text beschriebenen Verfahren werden die in der folgenden Tabelle genannten Verbindungen der Formel (I) hergestellt.

Tabelle

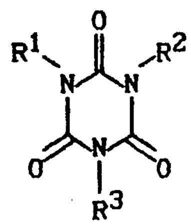

(I)

| Beispiel-Nummer | $R^3$ | $R^1$ | $R^2$ | Physikalische Konstanten |
|---|---|---|---|---|
| 1 | $Ph-CH_2-$ | $-C(CH_3)_3$ | $-C_2H_5$ | Kp. 170° C/0,03 mbar |
| 2 | $2-Cl-Ph-CH_2-$ | $-C(CH_3)_3$ | $-C_2H_5$ | $^1H$-NMR($CDCl_3$): 7,39m(1H), 7,22(1H) 7,06m(1H), 5,14s(2H), 3,97q(2H;J=7Hz), 1,67s(9H), 1,23t(3H;J=7Hz). |
| 3 | $4-Cl-Ph-CH_2-$ | $-C(CH_3)_3$ | $-C_2H_5$ | $^1H$-NMR($CDCl_3$): 7,40d(2H;J=8Hz) 7,27d(2H;J=8Hz), 4,95s(2H), 3,89q(2H;J=7Hz), 1,66s(9H), 1,21t(3H;J=7Hz). |
| 4 | $2,6-Cl_2-Ph-CH_2-$ | $-C(CH_3)_3$ | $-C_2H_5$ | Fp. < 50° C |
| 5 | $2,6-Cl_2-Ph-CH_2-$ | $-CH_2-C(CH_3)_3$ | $-CH_3$ | Fp. 130° C |
| 6 | $4-CH_3-Ph-CH_2-$ | $-C(CH_3)_3$ | $-C_2H_5$ | $^1H$-NMR($CDCl_3$): 7,36d(2H;J=8Hz), 7,13d(2H;J=8Hz), 4,96s(2H), 3,88q(2H;J=7Hz), 2,33s(3H), 1,66s(9H), 1,21t(3H;J=7Hz). |

T a b e l l e - Fortsetzung -

| Beispiel-Nummer | $R^3$ | $R^1$ | $R^2$ | Physikalische Konstanten |
|---|---|---|---|---|
| 7 | 4-$CH_3O$-Ph-$CH_2$- | -$C(CH_3)_3$ | -$C_2H_5$ | $^1$H-NMR($CDCl_3$): 7,42d(2H;J=8Hz), 6,85d(2H;J=8Hz), 4,94s(2H), 3,88q(2H;J=7Hz), 3,79s(3H), 1,65s(9H), 1,20t(3H;J=7Hz). |
| 8 | 4-$CF_3O$-Ph-$CH_2$- | -$C(CH_3)_3$ | -$C_2H_5$ | $^1$H-NMR($CDCl_3$): 7,51d(2H;J=8Hz), 7,17d(2H;J=8Hz), 4,99s(2H), 3,90q(2H;J=7Hz), 1,67s(9H), 1,22t(3H;J=7Hz). |
| 9 | 3,4-$(CH_3)_2$-Ph-$CH(CH_3)$- | -$C(CH_3)_3$ | -$CH_2$-CO-O-$C_2H_5$ | Fp. 72° C |
| 10 | 2,5-$(CH_3)_2$-Ph-$CH(CH_3)$- | -$C(CH_3)_3$ | -$C_2H_5$ | Öl |
| 11 | 2,4,5-$(CH_3)_3$-Ph-$CH(CH_3)$- | -$C(CH_3)_3$ | -$CH_2$-C≡CH | Fp. 122° C |
| 12 | 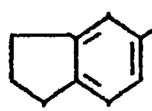 $CH(CH_3)$- | -$C(CH_3)_3$ | -$CH_2$-CH=$CH_2$ | |
| 13 | 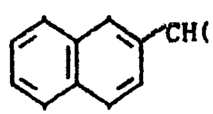 $CH(CH_3)$- | -$C(CH_3)_3$ | -$CH_2$-CN | Fp. 152° C |

T a b e l l e - Fortsetzung -

| Beispiel-Nummer | $R^3$ | $R^1$ | $R^2$ | Physikalische Konstanten |
|---|---|---|---|---|
| 14 | $3-CH_3-4-CH_3O-Ph-CH(CH_3)-$ | $-C(CH_3)_3$ | $-CH_3$ | |
| 15 | $3,4-(CH_3O)_2-Ph-CH(CH_3)-$ | $-C(CH_3)_3$ | $-C_5H_{11}$ | Öl |
| 16 | $Ph-CH_2-CH_2-$ | $-C(CH_3)_3$ | $-CH_3$ | Fp. 76°C |
| 17 | $Ph-CH_2-CH_2-$ | $-C(CH_3)_3$ | $-C_2H_5$ | Fp. 58°C |
| 18 | $Ph-CH_2-CH_2-$ | $-C(CH_3)_3$ | $-CH_2-C≡CH$ | $^1H$-NMR($CDCl_3$):7,33-7,19m(5H), 5,85ddt(1H;J=16+10+6Hz), 5,24dq(1H;J=16+1Hz), 5,21dq(1H;J=10+1Hz), 4,42dt(2H;J=6+1Hz), 4,07dd(2H;J=7+6Hz), 3,93dd(2H;J=7+6Hz), 1,61s(9H). |
| 19 | $Ph-CH_2-CH_2-$ | $-C(CH_3)_3$ | $-CH_2-CH=CH_2$ | Fp. 98°C |
| 20 | $Ph-CH_2-CH_2-$ | $-C(CH_3)_3$ | $-C_3H_7$ | $^1H$-NMR($CDl_3$):7,33-7,18m(5H), 4,07dd(2H;J=6+7Hz), 3,77dd(2H;J=6+7Hz), 2,92dd(2H;J=6+7Hz), 1,71-1,51m(2H) 1,63s(9H, 0,92t(3H;J=7Hz). |

T a b e l l e - Fortsetzung -

| Beispiel-Nummer | $R^3$ | $R^1$ | $R^2$ | Physikalische Konstanten |
|---|---|---|---|---|
| 21 | $Ph-CH_2-CH_2-$ | $-C(CH_3)_3$ | $-CH_2-CO-O-C_2H_5$ | Fp. 90° C |
| 22 | $Ph-CH(C_2H_5)-CH_2-$ | $-C(CH_3)_3$ | $-C_2H_5$ | $^1$H-NMR($CDCl_3$):7,30-7,12m(5H) 4.12dd(1H;J=10+14Hz), 3,93dd(1H;J=7+14Hz), 3,78q(2H;J=7Hz), 3,04ddt(1H;J=7+10+7Hz), 1,71 quint. (2H;J=7Hz), 1,54s(9H), 1,09t(3H;J=7Hz), 0,81t(3H;J=7Hz). |
| 23 | $Ph-CH(C_2H_5)-CH_2-$ | $-C(CH_3)_3$ | $-CH_2-CN$ | Fp. 95° C |
| 24 | $2-Cl-Ph-CH_2-CH_2-$ | $-C(CH_3)_3$ | $-C_5H_{11}$ | $^1$H-NMR($CDCl_3$):7,37-7,10m(4H), 4,15t(2H;J=7Hz), 3,73tbr(2H;J=8Hz), 3,09t(2H;J=7Hz), 1,61s(9H), 1,65-1,47m(2H), 1,35-1,20m(4H), 0,90t(3H;J=7Hz). |
| 25 | $2-Cl-Ph-CH(CH_3)-CH_2-$ | $-C(CH_3)_3$ | $-C_2H_5$ | $^1$H-NMR($CDCl_3$):7,40dbr(1H;J=8Hz), 7,30-7,19m(2H), 7,12dt(1H;J=2+8Hz), 4,18-3,83m(3H), 3,79q(2H;J=7Hz), 1,56s(9H), 1,32d(3H;J=7Hz), 1,09t(3H;J=7Hz). |

T a b e l l e - Fortsetzung -

| Beispiel-Nummer | $R^3$ | $R^1$ | $R^2$ | Physikalische Konstanten |
|---|---|---|---|---|
| 26 | 3-Cl-Ph-$CH_2$-$CH_2$- | -$C(CH_3)_3$ | -$C_2H_5$ | $^1$H-NMR($CDCl_3$):7,28-7,11m(4H), 4,04dd(2H;J=6+7Hz), 3,88q(2H;J=7Hz), 2,90dd(2H;J=6+7Hz), 1,64s(9H), 1,20t(3H;J=7Hz). |
| 27 | 4-Cl-Ph-$CH_2$-$CH_2$- | -$C(CH_3)_3$ | -$C_2H_5$ | $^1$H-NMR($CDCl_3$):7,26d(2H;J=8Hz), 7,17d(2H;J=8Hz), 4,02dd(2H;J=6+7Hz), 3,88q(2H;J=7Hz), 2,89dd(2H;J=6+7Hz), 1,63s(9H), 1,20t(3H;J=7Hz). |
| 28 | 4-Cl-Ph-$CH_2$-$CH_2$- | -$C(CH_3)_3$ | -$C_3H_7$ | Fp. 62°C |
| 29 | 4-Cl-Ph-CH-$CH_2$-<br>\|<br>$CH(CH_3)_2$ | -$C(CH_3)_3$ | -$CH_2$-C≡CH | $^1$H-NMR($CDCl_3$):7,20d(2H;J=8Hz), 7,05d(2H;J=8Hz), 4,47dd(2H;J=2+1Hz), 4,30dd(1H;J=10+12Hz), 4,01dd(1H;J=6+12Hz), 2,92ddd(1H;J=6+8+10Hz), 2,23t(1H;J=2Hz), 1,94d sept.(1H;J=8+7Hz), 1,51s(9H), 1,08d(3H;J=7Hz), 0,74d(3H;J=7Hz), |

T a b e l l e - Fortsetzung -

| Beispiel-Nummer | $R^3$ | $R^1$ | $R^2$ | Physikalische Konstanten |
|---|---|---|---|---|
| 30 | 2,4-$Cl_2$-Ph-$CH_2$-$CH_2$- | -$C(CH_3)_3$ | -$CH_3$ | Fp. 86° C |
| 31 | 2,4-$Cl_2$-Ph-$CH_2$-$CH_2$- | -$C(CH_3)_3$ | -$C_2H_5$ | $^1$H-NMR($CDCl_3$):7,36t(1H;J=1Hz), 7,17d(2H;J=1Hz), 4,11t(2H;J=7Hz), 3,85q(2H;J=7Hz), 3,06t(2H;J=7Hz), 1,61s(9H), 1,16t(3H;J=7Hz). |
| 32 | 3,4-$Cl_2$-Ph-$CH_2$-$CH_2$- | -$C(CH_3)_3$ | -$C_2H_5$ | $^1$H-NMR($CDCl_3$):7,37d(1H;J=8Hz), 7,33d(1H;J=2Hz), 7,10dd(1H;J=8+2Hz), 4,03dd(2H;J=6+7Hz), 3,88q(2H;J=7Hz), 2,88dd(2H;J=6+7Hz), 1,64 s(9H), 1,19t(3H;J=7Hz). |
| 33 | 3,4-$Cl_2$-Ph-$CH_2$-$CH_2$- | -$C(CH_3)_3$ | -$CH_2$-C≡CH | Fp. 88° C |
| 34 | 2,6-$Cl_2$-Ph-$CH_2$-$CH_2$- | -$C(CH_3)_3$ | -$C_2H_5$ | Fp. 112° C |
| 35 | 2,6-$Cl_2$-Ph-CH($CH_3$)-$CH_2$- | -$C(CH_3)_3$ | -$C_2H_5$ | Fp. 124° C |
| 36 | 2-$CH_3$-Ph-$CH_2$-$CH_2$- | -$C(CH_3)_3$ | -$C_2H_5$ | $^1$H-NMR($CDCl_3$): 7,19-7,09m(4H), 4,01dd(2H;J=6+7Hz), 3,88q(2H;J=7Hz), 2,93dd(2H;J=6+7Hz), 2,41s((3H), 1,64s(9H), 1,19t(3H;J=7Hz). |

**T a b e l l e - Fortsetzung -**

| Beispiel-Nummer | $R^3$ | $R^1$ | $R^2$ | Physikalische Konstanten |
|---|---|---|---|---|
| 37 | $3-CH_3-Ph-CH_2-CH_2-$ | $-C(CH_3)_3$ | $-C_2H_5$ | $^1H$-NMR($CDCl_3$):7,20t(1H;J=8Hz), 7,07-7,00m(3Hz), 4,05dd(2H;J=6+7Hz), 3,88q(2H;J=7Hz), 2,88dd(2H;J=6+7Hz), 2,31s(3H), 1,63s(9H), 1,19t(3H;J=7Hz). |
| 38 | $4-CH_3-Ph-CH_2-CH_2$ | $-C(CH_3)_3$ | $-C_2H_5$ | Fp. 64°C |
| 39 | $4-CH_3-Ph-CH_2-CH_2-$ | $-C(CH_3)_3$ | $-CH(CH_3)_2$ | $^1H$-NMR($CDCl_3$):7,17-7,07m(4H), 4,91 sept. (1H;J=7Hz), 4,01dd(2H;J=6+7Hz), 2,87dd(2H;J=6+7Hz), 2,30s(3H), 1,62s(9H, 1,41d(6H;J=7Hz). |
| 40 | $3-CF_3-Ph-CH_2-CH_2-$ | $-C(CH_3)_3$ | $-C_2H_5$ | Fp. 66°C |
| 41 | $3-CF_3-Ph-CH_2-CH_2-$ | $-C(CH_3)_3$ | $-CH(CH_3)_2$ | $^1H$-NMR($CDCl_3$):7,48-7,42m(4H), 4,91 sept. (1H;J=7Hz), 4,07dd(2H;J=6+7Hz), 3,00dd(2H;J=6+7Hz), 1,61s(9H), 1,40d(6H;J=7Hz). |
| 42 | $4-(CH_3)_3C-Ph-CH_2-CH_2-$ | $-C(CH_3)_3$ | $-CH_3$ | $^1H$-NMR(DMSO):7,30d(2H;J=8Hz), 7,11d(2H;J=8Hz), 3,90dd(2H;J=6+7Hz), 3,11s(3H), 2,79dd(2H;J=6+7Hz), 1,52s(9H), 1,25s(9H). |

Tabelle - Fortsetzung -

| Beispiel-Nummer | $R^3$ | $R^1$ | $R^2$ | Physikalische Konstanten |
|---|---|---|---|---|
| 43 | $4-(CH_3)_3C-Ph-CH_2-CH_2-$ | $-C(CH_3)_3$ | $-C_2H_5$ | $^1H$-NMR($CDCl_3$):7,32d(2H;J=8Hz), 7,17d(2H;J=8Hz) 4,07dd(2H;J=6+7Hz) 3,88q(2H;J=7Hz), 2,90dd(2H;J=6+7Hz), 1,61s(9H), 1,29s(9H), 1,18t(3H;J=7Hz). |
| 44 | $3,4-(CH_3)_2-Ph-CH_2-CH_2$ | $-C(CH_3)_3$ | $-C_2H_5$ | $^1H$-NMR($CDCl_3$):7,07d(1H;J=8Hz), 7,01d(1H;J=1Hz), 6,98dd(1H;J=8+1Hz), 4,01dd(2H;J=6+7Hz), 3,88q(2H;J=7Hz), 2,86dd(2H;J=6+7Hz), 2,23sbr(6H), 1,65s(9H), 1,19t(3H;J=7Hz). |
| 45 | $2-CH_3O-Ph-CH_2-CH_2-$ | $-C(CH_3)_3$ | $-C_2H_5$ | $^1H$-NMR(DMSO):7,18dt(1H;J=2+8Hz), 7,07dd(1H;J=2+8Hz), 6,91dbr(1H;J=8Hz), 6,84tbr(1H;J=8Hz), 3,97t(2H;J=7Hz), 3,74s(3H), 3,70q(2H;J=7Hz), 2,86t(2H;J=7Hz), 1,52s(9H), 1,06t(3H;J=7Hz). |
| 46 | $3-CH_3O-Ph-CH_2-CH_2-$ | $-C(CH_3)_3$ | $-C_2H_5$ | $^1H$-NMR($CDCl_3$):7,21dt(1H;J=2+8Hz), 6,87-6,73m(3H), 4,06dd(2H;J=6+7Hz), 3,88q(2H;J=7Hz), 3,78s(3H), 2,90dd(2H;J=6+7Hz), 1,63s(9H), 1,19t(3H;J=7Hz). |

T a b e l l e - Fortsetzung -

| Beispiel-Nummer | $R^3$ | $R^1$ | $R^2$ | Physikalische Konstanten |
|---|---|---|---|---|
| 47 | $4-CH_3O-Ph-CH_2-CH_2-$ | $-C(CH_3)_3$ | $-C_2H_5$ | $^1$H-NMR($CDCl_3$):7,16d(2H;J=8Hz), 6,83d(2H;J=8Hz) 4,02dd(2H;J=6+7Hz) 3,87q(2H;J=7Hz), 3,78s(3H), 2,87dd(2H;J=6+7Hz), 1,64s(9H), 1,19t(3H;J=7Hz). |
| 48 | $3-Cl-4-CF_3O-Ph-CH_2-CH_2-$ | $-C(CH_3)_3$ | $-C_2H_5$ | $^1$H-NMR($CDCl_3$):7,36d(1H;J=2Hz), 7,29-7,14m(2H), 4,05dd(2H;J=7+8Hz), 3,89q(2H;J=7Hz), 2,92dd(2H;J=7+8Hz), 1,63s(9H), 1,19t(3H;J=7Hz). |
| 49 | $2,5-(CH_3O)_2-Ph-CH_2-CH_2-$ | $-C(CH_3)_3$ | $-C_2H_5$ | $^1$H-NMR($CDCl_3$):6,78-6,67m(3H), 4,10t(2H;J=7Hz), 3,83t(2H;J=7Hz), 3,77s(3H), 2,91t(2H;J=7Hz), 1,60s(9H), 1,15t(3H;J=7Hz), 3,73s(3H). |
| 50 | $3,4-(CH_3O)_2-Ph-CH_2-CH_2-$ | $-C(CH_3)_3$ | $-C_2H_5$ | $^1$H-NMR(DMSO):6,86d(1H;J=8Hz), 6,74d(1H;J=2Hz), 6,68dd(1H;J=8+2Hz), 3,91dd(2H;J=6+7Hz), 3,71q(2H;J=7Hz), 3,72(3H), 3,70s(3H),2,77d(2H;J=6+7Hz), 1,54s(9H), 1,07t(3H;J=7Hz). |

Tabelle - Fortsetzung -

| Beispiel-Nummer | $R^3$ | $R^1$ | $R^2$ | Physikalische Konstanten |
|---|---|---|---|---|
| 51 | 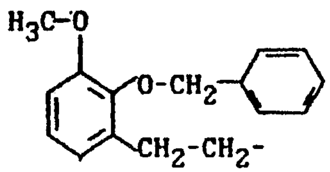 | $-C(CH_3)_3$ | $-C_2H_5$ | $^1$H-NMR($CDCl_3$):7,51dd(2H;J=8+2Hz), 7,41-7,29m(4H), 7,00t(1H;J=8Hz), 6,80dt(2H;J=2+8Hz), 5,01s(2H), 4,06t(2H;J=7Hz), 3,85s(3H), 3,75q(2H;J=7Hz), 2,91t(2H;J=7Hz), 1,58s(9H), 1,23t(3H;J=7Hz). |
| 52 | $3-(CH_3O)-Ph-CH(CH_3)CH_2-$ | $-C(CH_3)_3$ | $-C_2H_5$ | $^1$H-NMR($CDCl_3$):7,18(1H) 6,75(1H), 6,70(1H), 3,78s(3H), 3,70-4,20m(6H), 2,95-3,12m(1H), 1,55s(9H), 1,11t, (3H;J=7Hz), 0,82t(3H;J=7Hz). |
| 53 | $4-(CH_3O)-Ph-CH[CH(CH_3)_2]CH_2-$ | $-CH_2-C(CH_3)_3$ | $-C_2H_5$ | $^1$H-NMR($CDCl_3$):7,00d(2H;J=8Hz), 6,75d, (2H;J=8Hz), 4,33dd(1H;J=2Hz+16Hz), 4,05dd(1H;J=6Hz+16Hz), 3,80q(2H;J=7Hz), 3,75s(3H), 3,67s(2H), 2,80-3,00m(1H), 1,85-2,00m(1H), 1,09t(3H;J=7Hz), 0,80s(9H), 0,75d(6H;J=7Hz). |
| 54 | $2,6-Cl_2-Ph-(CH_2)_2CH(CH_3)-$ | $-C(CH_3)_3$ | $-C_2H_5$ | $^1$H-NMR($CDCl_3$):7,25(2H) 7,05dd(1H), 4,80-5,00m(1H), 3,88q(2H;J=7Hz), 2,70-3,00m(2H), 2,20-2,42m(1H), 1,90-2,15m(1H), 1,68s(9H), 1,50d(3H;J=7Hz), 1,22t(3H;J=7Hz). |

T a b e l l e - Fortsetzung -

| Beispiel-Nummer | $R^3$ | $R^1$ | $R^2$ | Physikalische Konstanten |
|---|---|---|---|---|
| 55 | 4-$(CH_3O)$-Ph-CH[CH$(CH_3)_2$]$CH_2$- | -C$(CH_3)_3$ | -$C_2H_5$ | $^1$H-NMR($CDCl_3$):7,05d(2H;J=8Hz), 6,78d (2H;J=8Hz), 4,38dd(1H;J=2+16Hz), 4,08dd(1H;J=6+16Hz), 3,81q(2H;J=7Hz), 3,69s(3H), 2,80-3,00m(1H), 1,90-2,10m(1H), 1,55s(9H), 1,10t (3H;J=7Hz), 0,75(6H;J=7Hz). |
| 56 | 3-$(CH_3O)$-Ph-CH[CH$(CH_3)_2$]$CH_2$- | -C$(CH_3)_3$ | -$C_2H_5$ | $^1$H-NMR($CDCl_3$):7,10dt(1H;J=1+5Hz), 6,65-6,70m(2H), 4,31dd(1H;J=2+16Hz), 3,98s(1H;J=16+5Hz), 3,76s(3H), 3,72q (2H;J=7Hz), 2,85-3,00m(1H), 1,85-2,05m(1H), 1,50s (9H), 1,08t(3H;J=7Hz), 0,78d(6H;J=7Hz). |
| 57 | Ph-$CH_2$-$CH_2$-CH$(CH_3)$- | -C$(CH_3)_3$ | -$C_2H_5$ | $^1$H-NMR($CDCl_3$):7,10-7,30m(5H),4,75-4,95m (1H), 3,89q(2H;J=7Hz), 2,35-2,80m(3H), 1,90-2,10m(1H), 1,65s(9H), 1,43d(3H;J=7Hz), 1,18t(3H;J=7Hz). |
| 58 | 4-$(CH_3O)$-Ph-CH$(CH_3)$- | -C$(CH_3)_3$ | -$C_2H_5$ | $^1$H-NMR($CDCl_3$):7,35d(2H;J=10Hz), 6,85d (2H;J=10Hz), 5,98q(1H;J=80Hz), 3,85q (2H;J=7Hz), 3,78s(3H), 1,82d(3H;J=8Hz), 1,60s(9H), 1,20t(3H;J=7Hz). |

T a b e l l e - Fortsetzung -

| Beispiel-Nummer | $R^3$ | $R^1$ | $R^2$ | Physikalische Konstanten |
|---|---|---|---|---|
| 59 | $Ph-C(CH_3)_2CH_2-$ | $-C(CH_3)_3$ | $-C_2H_5$ | $^1H$-NMR($CDCl_3$):7,15-7,45m(5H), 4,05s (2H), 3,85q(2H;J=7Hz), 1,60s(9H), 1,38s(6H), 1,18t(3H;J=7Hz). |
| 60 | $2-Cl-Ph-CH_2-CH_2-CH(CH_3)-$ | $-C(CH_3)_3$ | $-C_2H_5$ | $^1H$-NMR($CDCl_3$):7,05-7,25m(4H), 4,85tg(1H), 3,85q(2H;J=7Hz), 2,30-2,85m(3H), 1,90-2,10m (1H), 1,65s(9H), 1,45d(3H;J7Hz), 1,22t(3H;J=7Hz). |
| 61 | $4-Cl-Ph-CH_2-CH_2-CH(CH_3)-$ | $-C(CH_3)_3$ | $-C_2H_5$ | $^1H$-NMR($CDCl_3$):7,19d(2H);J=9Hz), 7,05d(2H;J=9Hz), 4,83tg(1H), 3,81q(2H;J=7Hz), 2,30-2,70m(3H), 1,90-2,10m(1H), 1,65s(9H), 1,42d(3H,J=8Hz), 1,20t(3H;J=7Hz). |
| 62 | $PH-CH_2-$ | $-C(CH_3)_2C{\equiv}CH$ | $-CH_3$ | $^1H$-NMR($CDCl_3$):7,5m(2H), 7,25-7,40m(3H), 5,02s(2H), 3,28s(3H), 2,55s(1H), 1,95s(6H). |
| 63 | $Ph-CH_2-$ | $-C(CH_3)_2CH=CH_2$ | $-C_2H_5$ | |
| 64 | $Ph-CH_2-$ | 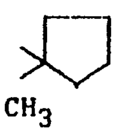 | $-C_2H_5$ | Fp. 52° C |

T a b e l l e - Fortsetzung -

| Beispiel-Nummer | $R^3$ | $R^1$ | $R^2$ | Physikalische Konstanten |
|---|---|---|---|---|
| 65 | 2-Cl-Ph-$CH_2$-$CH_2$- | -$C(CH_3)_3$ | -$C_2H_5$ | $^1$H-NMR($CDCl_3$):7,37-7,30m(1H), 7,24-7,13m(3H), 4,14t(2H;J=7Hz), 3,85q(2H;J=7Hz), 3,09t(2H;J=7Hz), 1,60s(9H), 1,14t(3H;J=7Hz). |
| 66 | 4-Cl-Ph-CH(CH($CH_3$)$_2$)-$CH_2$- | -$C(CH_3)_3$ | -$C_2H_5$ | $^1$H-NMR($CDCl_3$):7,21d(2H;J=8Hz), 7,04d(2H;J=8Hz), 4,28dd(1H;J=11+13Hz), 3,98dd(1H;J=6+13Hz), 3,75q(2H;J=7Hz), 2,92ddd(1H;J=6+9+11Hz), 1,94d sept.(1H;J=6+7Hz), 1,51s(9H), 1,06t (3H;J=7Hz), 1,08d(3H;J=7Hz), 0,74d(3H;J=7Hz). |

Es bedeuten:

Kp. = Siedepunkt
Fp. = Schmelzpunkt
Ph = Phenylring

Herstellung des noch neuen Harnstoffs = Ausgangsprodukt

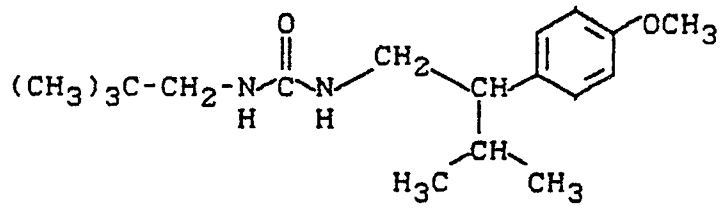

10,5 g (0,054 Mol) 2-(4-Methoxyphenyl)-2-(2-propyl)ethylamin werden in 100 ml absolutem Toluol gelöst und bei Raumtemperatur mit einer Lösung von 7,3 g (0,065 Mol) Neopentylisocyanat gelöst in 10 ml absolutem Toluol versetzt. Man kocht 15 min unter Rückfluß, läßt abkühlen und saugt den Niederschlag ab. Ausbeute 13,7 g = 83 % der Theorie mit einem Schmelzpunkt von 132 °C.

Analog diesem Beispiel oder den angegebenen Literaturstellen können die anderen Harnstoffderivate der Formeln (VI) und (VII) hergestellt werden.

Anwendungsbeispiele

In den folgenden Anwendungsbeispielen werden die nachstehend aufgeführten Verbindungen als Vergleichssubstanzen eingesetzt:

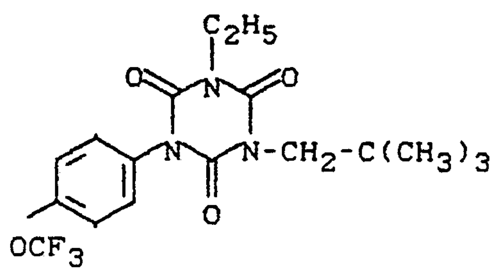

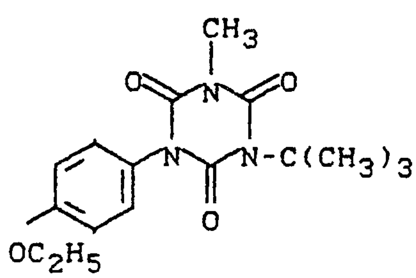

Beispiel A

Pyricularia-Test (Reis) / protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gebenüber dem Stand der Technik zeigen in diesem Test viele der erfindungsgemäßen Verbindungen.

Beispiel B

Pyricularia-Test (Reis) systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Die meisten der Verbindungen zeigen z.B. bei einer Aufwandmenge von 100 mg Wirkstoff pro 100 $cm^2$ einen guten Wirkungsgrad.

## Ansprüche

1. Trisubstituierte 1,3,5-Triazin-2,4,6-trione der allgemeinen Formel (I)

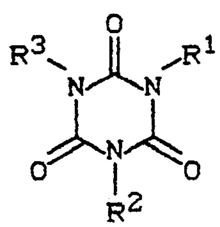

(I)

in welcher
$R^1$, $R^2$ und $R^3$ unterschiedliche Reste sind und
$R^1$ für ein gegebenenfalls substituierten aliphatischen oder cycloaliphatischen Rest steht,
$R^2$ für einen gegebenenfalls substituierten aliphatischen Rest steht und
$R^3$ für ein gegebenenfalls substituiertes Aralkyl steht.

2. Trisubstituierte 1,3,5-Triazin-2,4,6-trione gemäß Anspruch 1 der Formel (I),
in welcher
$R^1$, $R^2$ und $R^3$ unterschiedliche Reste sind und
$R^1$ für geradkettiges oder verzweigtes Alkyl mit 1 bis 12 Kohlenstoffatomen, geradkettiges oder verzweigtes Alkenyl mit 3 bis 12 Kohlenstoffatomen oder geradkettiges oder verzweigtes Alkinyl mit 3 bis 12 Kohlenstoffatomen steht, welche gegebenenfalls ein- bis mehrfach, gleich oder verschieden substituiert sind durch Alkoxy mit 1 bis 4 Kohlenstoffatomen, Halogenalkoxy mit 1 bis 3 Kohlenstoffatomen und 1 bis 5 gleichen oder verschiedenen Halogenatomen, Alkylthio mit 1 bis 3 Kohlenstoffatomen, Halogenalkylthio mit 1 bis 3

Kohlenstoffatomen und 1 bis 5 gleichen oder verschiedenen Halogenatomen; weiterhin $R^1$ für Cycloalkyl mit 3 bis 8 Kohlenstoffatomen steht, welches gegebenenfalls ein- bis sechsfach, gleich oder verschieden substituiert ist durch Alkyl mit 1 bis 4 Kohlenstoffatomen, Alkoxy mit 1 bis 3 Kohlenstoffatomen, Halogenalkoxy mit 1 bis 3 Kohlenstoffatomen und 1 bis 5 gleichen oder verschiedenen Halogenatomen, Alkylthio mit 1 bis 3 Kohlenstoffatomen und Halogenalkylthio mit 1 bis 3 Kohlenstoffatomen und 1 bis 5 gleichen oder verschiedenen Halogenatomen,

$R^2$ für Alkyl mit 1 bis 10 Kohlenstoffatomen, Alkenyl mit 3 bis 10 Kohlenstoffatomen, Alkinyl mit 3 bis 10 Kohlenstoffatomen, Alkoxyalkyl mit je 1 bis 3 Kohlenstoffatomen im Alkoxyteil und im Alkylteil, Alkylthioalkyl mit je 1 bis 3 Kohlenstoffatomen im Alkylthioteil und im Alkylteil, Alkoxycarbonylalkyl mit 1 bis 3 Kohlenstoffatomen im Alkoxyteil und 2 oder 3 Kohlenstoffatomen im Alkylteil oder für Cyanoalkyl mit 1 bis 5 Kohlenstoffatomen im Alkylteil steht,

$R^3$ für Aralkyl mit 1 bis 12 Kohlenstoffatomen im geradkettigen oder verzweigten Alkylteil und 6 bis 12 Kohlenstoffatomen im Arylteil steht, wobei der Arylrest gegebenenfalls ein- bis fünffach, gleich oder verschieden substituiert ist durch Halogen, Alkyl mit 1 bis 6 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Monoalkyl-oder Dialkylamino mit gegebenenfalls gleichen oder verschiedenen, geradkettigen oder verzweigten Alkylresten mit je 1 bis 4 Kohlenstoffatomen, Nitro und/oder Benzyloxy oder der Arylrest gegebenenfalls einen ankondensierten, gesättigten Ring mit 3 bis 5 Kohlenstoffatomen trägt.

3. Trisubstituierte 1,3,5-Triazin-2,4,6-trione gemäß Anspruch 1 der Formel (I),
in welcher
$R^1$, $R^2$ und $R^3$ unterschiedliche Reste sind und
$R^1$ für geradkettiges oder verzweigtes Alkyl mit 1 bis 10 Kohlenstoffatomen, geradkettiges oder verzweigtes Alkenyl mit 3 bis 8 Kohlenstoffatomen oder geradkettiges oder verzweigtes Alkinyl mit 3 bis 8 Kohlenstoffatomen steht, welche jeweils gegebenenfalls ein- bis dreifach, gleich oder verschieden substituiert sind durch Alkoxy mit 1 bis 3 Kohlenstoffatomen, Halogenalkoxy mit 1 oder 2 Kohlenstoffatomen und 1 bis 3 Chlor- und/oder Fluoratomen, Alkylthio mit 1 oder 2 Kohlenstoffatomen, Halogenalkylthio mit 1 oder 2 Kohlenstoffatomen und 1 bis 3 Chlor- und/oder Fluoratomen; $R^1$ weiterhin für Cycloalkyl mit 3 bis 6 Kohlenstoffatomen steht, welches gegebenenfalls ein- bis sechsfach, gleich oder verschieden substituiert ist durch Fluor, Chlor, Alkyl mit 1 bis 3 Kohlenstoffatomen, Alkoxy mit 1 oder 2 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen und Halogenalkylthio mit 1 oder 2 Kohlenstoffatomen und 1 bis 3 Chlor- und/oder Fluoratomen,

$R^2$ für Alkyl mit 1 bis 8 Kohlenstoffatomen, Alkenyl mit 3 bis 8 Kohlenstoffatomen, Alkinyl mit 3 bis 8 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht und

$R^3$ für Aralkyl mit 1 bis 5 Kohlenstoffatomen im geradkettigen oder verzweigten Alkylteil und Aryl für Phenyl oder Naphthyl steht, welche gegebenenfalls ein- bis vierfach, gleich oder verschieden substituiert sein können durch Fluor, Chlor, Brom, Alkyl mit 1 bis 5 Kohlenstoffatomen, Halogenalkyl mit 1 oder 2 Kohlenstoffatomen und 1 bis 5 Chlor- und/ oder Fluoratomen, Alkoxy mit 1 bis 3 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und 1 bis 5 Chlor- und/oder Fluoratomen, Dialkylamino mit gleichen oder verschiedenen, geradkettigen oder verzweigten Alkylresten mit je 1 bis 3 Kohlenstoffatomen, Nitro und/oder Benzyloxy oder der Phenylring in 1,2-Stellung eine Tri- oder Tetramethylenkette enthält.

4. Trisubstituierte 1,3,5-Triazin-2,4,6-trione gemäß Anspruch 1 der Formel (I),
in welcher
$R^1$, $R^2$ und $R^3$ unterschiedliche Reste sind und
$R^1$ für geradkettiges oder verzweigtes Alkyl mit 1 bis 8 Kohlenstoffatomen, geradkettiges oder verzweigtes Alkenyl mit 3 bis 6 Kohlenstoffatomen oder geradkettiges oder verzweigtes Alkinyl mit 3 bis 6 Kohlenstoffatomen steht, welche ein- bis dreifach, gleich oder verschieden substituiert sein können durch Alkoxy mit 1 oder 2 Kohlenstoffatomen, Alkylthio mit 1 oder 2 Kohlenstoffatomen; oder $R^1$ weiterhin für Cycloalkyl mit 3 bis 6 Kohlenstoffatomen steht, welches gegebenenfalls ein- bis dreifach, gleich oder verschieden durch Fluor, Chlor, Methyl, Ethyl, Methoxy, Ethoxy, Methylthio, Ethylthio und/ oder Trifluormethyl substituiert sein

kann,
$R^2$ für Methyl, Ethyl, Propyl, n-, sec.-, iso-, tert.-Butyl, n-Pentyl, iso-Pentyl, neo-Pentyl, tert.-Pentyl, Allyl, 2-Butenyl, Propargyl, 2-Butinyl, Methoxymethyl, Ethoxymethyl, Methylthiomethyl, Methoxycarbonylmethyl, Ethoxycarbonylmethyl, Methoxycarbonylethyl, Cyanmethyl oder Cyanethyl steht und
$R^3$ für Aralkyl mit 1 bis 7 Kohlenstoffatomen im geradkettigen oder verzweigten Alkylrest und Aryl für Phenyl oder Naphthyl steht, welche ein- bis dreifach, gleich oder verschieden substituiert sein können durch Fluor, Chlor, Alkyl mit 1 bis 4 Kohlenstoffatomen, Halogenalkyl mit 1 oder 2 Kohlenstoffatomen und 1 bis 5 Chlor- und/ oder Fluoratomen, Alkoxy mit 1 oder 2 Kohlenstoffatomen, Halogenalkoxy mit 1 oder 2 Kohlenstoffatomen und 1 bis 5 Chlor-und/oder Fluoratomen, Benzyloxy oder der Phenylring in 1,2-Stellung eine Trimethylenkette trägt.
5. Verfahren zur Herstellung von trisubstituierten 1,3,5-Triazin-2,4,6-trionen der Formel (I),

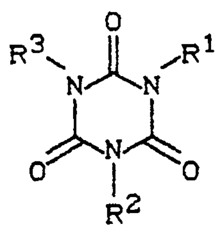
(I)

in welcher
$R^1$, $R^2$ und $R^3$ unterschiedliche Reste sind und
$R^1$ für einen gegebenenfalls substituierten aliphatischen oder cycloaliphatischen Rest steht,
$R^2$ für einen gegebenenfalls substituierten aliphatischen Rest steht und
$R^3$ für ein gegebenenfalls substituiertes Aralkyl steht, dadurch gekennzeichnet, daß man
a) 1,3-disubstituierte 1,3,5-Triazin-2,4,6-trione der allgemeinen Formel (II)

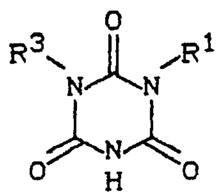
(II)

in welcher
$R^1$ und $R^3$ die oben angegebenen Bedeutungen haben, mit Verbindungen der allgemeinen Formel (III)
$R^2$-X (III)
in welcher
$R^2$ die oben angegebene Bedeutung hat und
X eine Abgangsgruppe bedeutet,
gegebenenfalls in Gegenwart eines Verdünnungs-oder Lösungsmittel und gegebenenfalls in Gegenwart eines Säurebindemittels umsetzt oder
1,3-disubstituierte 1,3,5-Triazin-2,4,6-trione der allgemeinen Formel (IV)

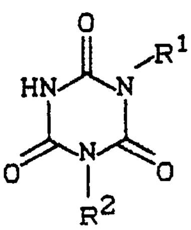
(IV)

in welcher
$R^1$ und $R^2$ die oben angegebene Bedeutungen haben, mit Verbindungen der allgemeinen Formel (V)

$R^3$-X (V)

in welcher

$R^3$ und X die oben angegebene Bedeutung haben

gegebenenfalls in Gegenwart eines Verdünnungs-oder Lösungsmittels umsetzt oder

c) N,N′-disubstituierte Harnstoffe der allgemeinen Formel (VI)

$R^1$-NH-CO-NH-$R^3$ (VI)

in welcher

$R^1$ und $R^3$ die oben angegebenen Bedeutungen haben, mit einem Bischlorcarbonylamin der allgemeinen Formel (VII)

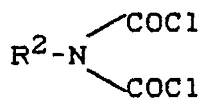

(VII)

in welcher

$R^2$ die oben angegebenen Bedeutung hat,

gegebenenfalls in Gegenwart eines Verdünnungs-oder Lösungsmittel und gegebenenfalls in Gegenwart eines Säurebindemittels umsetzt oder

d) N,N′-disubstituierte Harnstoffe der allgemeinen Formel (VIII)

$R^1$-NH-CO-NH-$R^2$ (VIII)

in welcher

$R^1$ und $R^2$ die oben angegebene Bedeutung haben, mit Verbindungen der allgemeinen Formel (IX)

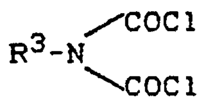

(IX)

in welcher

$R^3$ die oben angegebene Bedeutung hat, gegebenenfalls in Gegenwart eines Verdünnungs- oder Lösungsmittels und gegebenenfalls in Gegenwart eines Säurebindemittels,

umsetzt.

6. Schädlingsbekampfungsmittel, gekennzeichnet durch einen Gehalt an mindestens einem trisubstituierten 1,3,5-Triazin-2,4,6-trion der Formel (I) nach den Ansprüchen 1 und 5.

7. Verwendung von trisubstituierten 1,3,5-Triazin-2,4,6-trionen der Formel (I) nach den Ansprüchen 1 und 5 zur Bekämpfung von Schädlingen.

8. Verfahren zur Bekämpfung von Schädlingen, dadurch gekennzeichnet, daß man mindestens ein trisubstituiertes 1,3,5-Triazin-2,4,6-trion der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man mindestens ein trisubstituiertes 1,3,5-Triazin-2,4,6-trion der Formel (I) nach den Ansprüchen 1 und 5 mit Streckmitteln und/oder oberflächenaktiven Mitteln vermischt.

10. N-Neopentyl-N′-2-(4-methoxyphenyl)-2-(2-isopropyl)ethyl-harnstoff der Formel